# EUROPÄISCHE PATENTSCHRIFT

(11) **EP 0 693 475 B1**
(45) Veröffentlichungstag und Bekanntmachung des Hinweises auf die Patenterteilung: **11.02.1998**
(21) Anmeldenummer: 95110864.6
(22) Anmeldetag: 12.07.1995
(51) Int. Cl.: C07C 217/72, C07C 215/54, C07C 215/62, C07C 215/30, C07C 217/74, C07C 219/22, C07C 271/58, C07C 323/32, C07D 319/18, C07D 307/79, A61K 31/135

(54) **1-Phenyl-3-dimethylamino-propanverbindungen mit pharmakologischer Wirkung**
1-Phenyl-3-dimethylamino-propane derivatives having pharmacological activity
Dérivés propane 1-phényl-3-diméthylamino à activité pharmocologique

(30) Priorität: 23.07.1994 DE 4426245
(43) Veröffentlichungstag der Anmeldung: 24.01.1996
(73) Patentinhaber: Grünenthal GmbH, D-52078 Aachen (DE)
(72) Erfinder: Buschmann, Helmut, Dr., D-52066 Aachen (DE); Strassburger, Wolfgang, Prof. Dr., D-52146 Würselen (DE); Friderichs, Elmar, Dr., D-52223 Stolberg (DE)

(56) Entgegenhaltungen:
- EP-A- 0 176 049
- DD-A- 124 521
- CHEMICAL ABSTRACTS, vol. 54, no. 20, 25.Oktober 1960 Columbus, Ohio, US; abstract no. 20963c, I.N. NAZAROV ET AL. 'Synthetic analgesic substances.' Seite 20963; Spalte 1;
- JOURNAL OF PHARMACEUTICAL SCIENCES, Bd. 57, Nr. 9, September 1968 Seiten 1487-1493, N.D. POTTI ET AL. 'Use of 3-Azabicyclo(3.2.1)octane in the Mannich Reaction'
- JOURNAL OF PHAMACEUTICAL SCIENCES, Bd. 59, Nr. 7, Juli 1970 Seiten 1038-1041, PYARE PARIMOO ET AL. 'New Compounds: Some potential chemotherapeutic agents derived from aralkyl ketones'

## Beschreibung

Die Erfindung betrifft 1-Phenyl-3-dimethylamino-propanverbindungen, Verfahren zu deren Herstellung sowie die Verwendung dieser Substanzen als pharmazeutische Wirkstoffe.

Die Behandlung chronischer und nichtchronischer Schmerzzustände hat in der Medizin eine große Bedeutung. Dies spiegelt sich in der Vielzahl an Veröffentlichungen wieder. So sind beispielsweise aus EP 176 049 1-Naphthyl-3-amino-propan-1-ole mit analgetisch-narkotischer Wirkung bekannt. Sekundäre und tertiäre Alkohole mit γ-ständiger Aminogruppe sind in J. Pharm. Sci. 59, 1038 (1970) und J. Prakt. Chem. 323, 793 (1981), Phenyl-dimethylamino-propanole mit parasubstituiertem Phenylrest in Chem. Abstr. 54, 20963c (1960) und Chem. Abstr. 63, 6912e (1965) beschrieben. Auch diese Verbindungen besitzen analgetische Eigenschaften. Demgegenüber wirken die in DE 32 42 922 beschriebenen 3-Dimethylamino-propan-1-ole mit 2 Phenylresten antidepressiv. Die in J. Pharm. Sci. 57, 1487 (1968) beschriebenen 1-Phenyl-propan-1-ole haben in Abhängigkeit vom γständigen Azacyclus unterschiedliche pharmakologische Wirkungen.

Opioide werden seit vielen Jahren als Analgetika zur Schmerzbehandlung eingesetzt, obwohl sie eine Reihe von Nebenwirkungen, beispielsweise Sucht und Abhängigkeit, Atemdepression, gastro-intestinale Hemmwirkung und Obstipation, hervorrufen. Sie können daher nur unter besonderen Vorsichtsmaßnahmen wie speziellen Verordnungsvorschriften über einen längeren Zeitraum oder in höheren Dosierungen gegeben werden (Goodman, Gilman in "The Pharmacological Basis of Therapeutics", Pergaman Press, New York (1990)).

Tramadolhydrochlorid - (1RS,2RS)-2-[(Dimethylamino)methyl]-1-(3-methoxyphenyl)cyclohexanol, Hydrochlorid - nimmt unter den zentralwirksamen Analgetika eine Sonderstellung ein, da dieser Wirkstoff eine starke Schmerzhemmung ohne die für Opioide bekannten Nebenwirkungen hervorruft (J. Pharmacol. Exptl. Ther. 267, 331 (1993)). Tramadol ist ein Racemat und besteht aus gleichen Mengen an (+)- und (-)-Enantiomer. In vivo bildet der Wirkstoff den Metaboliten O-Desmethyl-tramadol, der gleichfalls als Enantiomerengemisch vorliegt. Untersuchungen haben ergeben, daß sowohl die Enantiomeren von Tramadol als auch die Enantiomeren der Tramadolmetabolite an der analgetischen Wirkung beteiligt sind (J. Pharmacol. Exp. Ther. 260, 275 (1992)).

Die der Erfindung zugrundeliegende Aufgabe bestand in der Entwicklung von analgetisch wirksamen Substanzen, die sich zur Behandlung starker Schmerzen eignen, ohne die für Opioide typischen Nebenwirkungen hervorzurufen. Darüber hinaus sollten die zu entwickelnden Substanzen nicht die während der Behandlung mit Tramadol in manchen Fällen auftretenden Nebenwirkungen, beispielsweise Übelkeit und Erbrechen, besitzen.

Es wurde gefunden, daß die an die zu entwickelnden Substanzen gestellten hohen Anforderungen von bestimmten 1-Phenyl-3-dimethylamino-propanverbindungen erfüllt werden. Diese Substanzen zeichnen sich durch eine ausgeprägte analgetische Wirkung aus, die im Vergleich zu Tramadol deutlich verstärkt ist.

Gegenstand der Erfindung sind dementsprechend 1-Phenyl-3-dimethylamino-propanverbindungen der Formel I 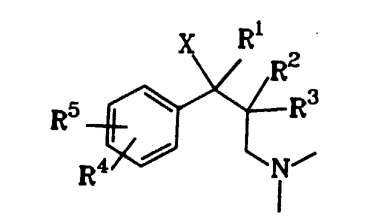 worin
X OH, F, Cl, H oder eine OCOR⁶-Gruppe mit R⁶ C₁₋₃-Alkyl bedeutet,
R¹ eine C₁₋₄-Alkylgruppe ist,
R² H oder eine C₁₋₄-Alkylgruppe und R³ H oder eine geradkettige C₁₋₄-Alkylgruppe bedeuten oder R² und R³ zusammen einen C₄₋₇-Cycloalkylrest darstellen, und
wenn R⁵ H ist, R⁴ meta-O-Z bedeutet mit Z H, C₁₋₃-Alkyl, PO(OC₁₋₄-Alkyl)₂, CO(OC₁₋₅-Alkyl),CONH-C₆H₄-(C₁₋₃-Alkyl), CO-C₆H₄-R⁷, wobei R⁷ ortho-OCOC₁₋₃-Alkyl oder meta- oder para-CH₂N(R⁸)₂ mit R⁸ C₁₋₄-Alkyl oder 4-Morpholino ist, oder R⁴ meta-S-C₁₋₃-Alkyl, meta-Cl, meta-F, meta-CR⁹R¹⁰R¹¹ mit R⁹, R¹⁰ und R¹¹ H oder F, ortho-OH, ortho-O-C₂-₃-Alkyl para-F oder para-CR⁹R¹⁰R¹¹ bedeutet, oder
wenn R⁵ para-ständiges Cl, F, OH oder O-C₁₋₃-Alkyl bedeutet, R⁴ meta-ständiges Cl, F, OH oder O-C₁₋₃-Alkyl bedeutet, oder
R⁴ und R⁵ zusammen 3,4-OCH=CH- oder 3,4-OCH=CHO- bedeuten,
als Diastereomere oder Enantiomere in Form ihrer Basen oder Salze von physiologisch verträglichen Säuren.

Bevorzugt werden 1-Phenyl-3-dimethylamino-propanverbindungen der Formel I, in denen X OH, F, Cl oder H darstellt, R¹ eine C₁₋₄-Alkylgruppe, R² H oder CH₃ und R³ H oder CH₃ bedeuten und wenn R⁵ H ist, R⁴ meta-ständiges OC₁₋₃-Alkyl, OH, S-C₁₋₃-Alkyl, F, Cl, CH₃, CF₂H oder CF₃, oder para-ständiges CF₃ bedeutet oder
wenn R⁵ ein para-ständiges Cl oder F ist, R⁴ meta-ständiges Cl oder F bedeutet, oder
R⁴ und R⁵ zusammen 3,4-OCH=CH- bedeuten.

Besonders bevorzugt werden 1-Phenyl-3-dimethylamino-propanverbindungen der Formel I, in der die Reste R² und R³ unterschiedliche Bedeutungen haben, in Form ihrer Diastereomeren mit der Konfiguration Ia 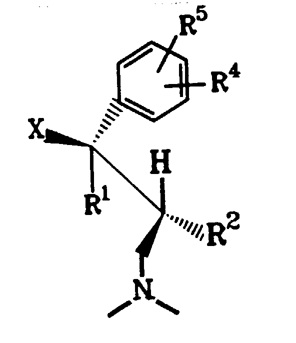 Weiterer Erfindungsgegenstand ist ein Verfahren zur Herstellung von 1-Phenyl-3-dimethylamino-propanverbindungen der Formel I, worin die Variable X OH bedeutet, welches dadurch gekennzeichnet ist, daß man ein β-Dimethylaminoketon der Formel II 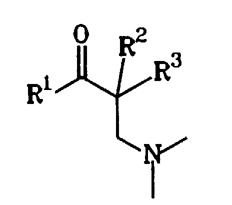 mit einer metallorganischen Verbindungen der Formel III 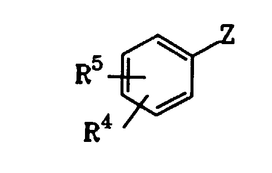 in der Z MgCl, MgBr, MgJ oder Li bedeutet, zu einer Verbindung der Formel I mit X = OH umsetzt.

Die Umsetzung eines β-Dimethylaminoketons mit einer Grignardverbindung der Formel III, in der Z MgCl, MgBr oder MgJ bedeutet, oder mit einer lithiumorganischen Verbindung der Formel III kann in einem aliphatischen Ether, beispielsweise Diethylether und/oder Tetrahydrofuran, bei Temperaturen zwischen -70° C und +60° C durchgeführt werden. Lithiumorganische Verbindungen der Formel III lassen sich durch Umsetzung einer Verbindung der Formel III, in der Z Cl, Br oder J bedeutet, mit beispielsweise einer n-Butyllithium/Hexan-Lösung durch Halogen-Lithium-Austausch erhalten. β-Dimethylaminoketone der Formel II können aus den Ketonen der allgemeinen Formel IV 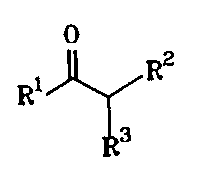 durch Umsetzung mit Dimethylaminhydrochlorid und Formaldehyd in Eisessig oder in einem C₁₋₄-Alkylalkohol oder durch Umsetzung mit Dimethylammoniummethylenchlorid in Acetonitril unter Acetylchlorid-Katalyse erhalten werden (Synthesis 1973, 703).

Bei der Umsetzung eines β-Dimethylaminoketons der Formel II, in der die Variablen R² und R³ unterschiedliche Bedeutungen haben, mit einer metallorganischen Verbindung der Formel III werden 1-Phenyl-3-dimethylamino-propanverbindungen der Formel I mit der relativen Konfiguration der Formel Ia 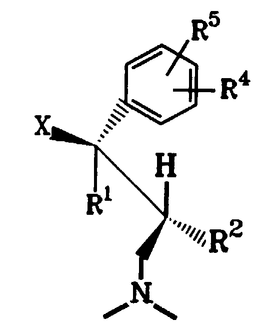 erhalten, in der X und die Dimethylaminomethylgruppe threo zueinander angeordnet sind. Würde dagegen die Umsetzung nach dem in DD 124 521 offenbarten Verfahren zur Herstellung von 1-Phenyl-1-hydroxy-3-aminopropanen durchgeführt werden, d. h. β-Aminoketone der Formel V 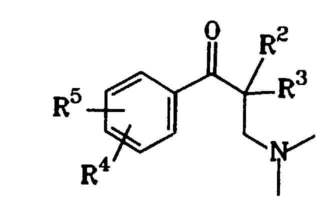 mit einer Alkylgrignardverbindung R¹MgHal umgesetzt werden, würden Verbindungen mit der relativen Konfiguration Ib 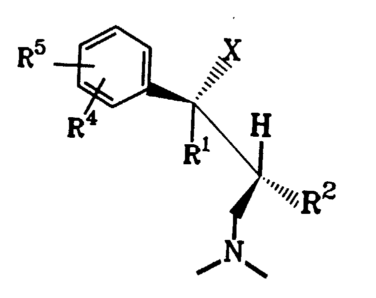 entstehen, in der die OH-Gruppe und der Dimethylaminomethylrest erythro zueinander angeordnet sind.

1-Phenyl-3-dimethylamino-propanverbindungen der Formel I, in denen R⁴ und/oder R⁵ die OH-Gruppe darstellen, lassen sich aus den entsprechenden 1-(4(5)-Methoxyphenyl)-3-dimethylamino-propanolverbindungen durch selektive Etherspaltung mit Diisobutylaluminiumhydrid in einem aromatischen Kohlenwasserstoff, beispielsweise Toluol, bei einer Temperatur zwischen 60 und 130° C herstellen (Synthesis 1975, 617).

Weiterer Erfindungsgegenstand ist ein Verfahren zur Herstellung von 1-Phenyl-3-dimethylamino-propanverbindungen der Formel I, in der X H bedeutet, welches dadurch gekennzeichnet ist, daß man eine Verbindung der Formel I, in der X Cl bedeutet, mit Zinkborhydrid, Zinkcyanoborhydrid und/oder Zinncyanoborhydrid umsetzt.

Üblicherweise wird die Reaktion in einem Lösungsmittel, beispielsweise Diethylether und/oder Tetrahydrofuran bei einer Temperatur zwischen 0° C und 30° C durchgeführt.

Verbindungen der Formel I, in der X H ist und R⁴ und/oder R⁵ die OH-Gruppe darstellen, lassen sich aus den entsprechenden Methoxyphenyl-Verbindungen durch mehrstündiges Erhitzen mit konzentrierter Bromwasserstoffsäure erhalten (Chem. Rev. 54, 615 (1954); J. Am. Chem. Soc. 74, 1316 (1952)).

Desweiteren ist Erfindungsgegenstand ein Verfahren zur Herstellung von 1-Phenyl-3-dimethylamino-propanverbindungen der Formel I, worin X F bedeutet, welches dadurch gekennzeichnet ist, daß man eine Verbindung der Formel I, in der X OH bedeutet, mit Dimethylamino-schwefeltrifluonid in einem Lösungsmittel umsetzt.

Als Lösungsmittel eignen sich Dichlormethan, 1,1,2-Trichlorethan und/oder Toluol. Üblicherweise wird die Reaktion bei einer Temperatur zwischen -50° C und +30° C durchgeführt (Org. React. 35, 513 (1988)). Wird eine Verbindung der Formel I mit X = OH eingesetzt, in der R⁴ und/oder R⁵ OH-Gruppen darstellen, müssen diese OH-Gruppen vor der Umsetzung mit der Fluorverbindung beispielsweise durch Umsetzung mit Benzoylchlorid geschützt werden.

Ferner ist Erfindungsgegenstand ein Verfahren zur Herstellung von 1-Phenyl-3-dimethylamino-propanverbindungen der Formel I, in der X Cl bedeutet, welches dadurch gekennzeichnet ist, daß man eine Verbindung der Formel I, in der X OH bedeutet, mit Thionylchlorid umsetzt.

Üblicherweise wird die Umsetzung in Abwesenheit eines Lösungsmittels bei einer Temperatur zwischen 0° C und 20° C durchgeführt. Der OH/Cl- Austausch erfolgt unter Erhalt der Konfiguration.

Weiterer Erfindungsgegenstand ist ein Verfahren zur Herstellung von 1-Phenyl-3-dimethylamino-propanverbindungen der Formel I, in der X eine OCOR⁶-Gruppe mit R⁶ C₁₋₃-Alkyl bedeutet, welches dadurch gekennzeichnet ist, daß man eine Verbindung der Formel I, in der X OH bedeutet, mit einem Säurechlorid Cl-COOR⁶ umsetzt.

Vorzugsweise wird die Reaktion in einem Lösungsmittel, beispielsweise Dichlormethan, Toluol und/oder Tetrahydrofuran, bei einer Temperatur zwischen -10° C und +30° C durchgeführt.

1-Phenyl-3-dimethylamino-propanverbindungen der Formel I, in denen R⁵ H ist und R⁴ eine meta-ständige Phosphat-, Carbonat-, Carbamat- oder Carboxylatgruppe darstellt, lassen sich durch Umsetzung der entsprechenden 1-(3-Hydroxyphenyl)-3-dimethylamino-propanverbindungen der Formel I in Form ihrer Alkalisalze mit einem Alkalisalz eines Dialkylchlorphosphates, mit einem Alkylchloroformiat, mit einem Arylisocyanat oder mit einem Carbonsäurechlorid erhalten. Diese Umsetzungen werden üblicherweise in einem Lösungsmittel, beispielsweise Toluol, Dichlormethan, Diethylether und/oder Tetrahydrofuran bei Temperaturen zwischen -15° C und +110° C durchgeführt (Drugs of the Future 16, 443 (1991); J. Med. Chem. 30, 2008 (1987) und 32, 2503 (1989); J. Org. Chem. 43, 4797 (1978); Tetrahedron Lett. 1977, 1571; J. Pharm. Sci. 57, 774 (1968)).

Die Verbindungen der Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in an sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in wäßriger Lösung.

1-Phenyl-3-dimethylamino-propanverbindungen der Formel I sind toxikologisch unbedenklich, so daß sie sich als pharamazeutischer Wirkstoff in Arzneimitteln eignen.

Weiterer Erfindungsgegenstand ist dementsprechend die Verwendung einer 1-Phenyl-3-dimethylaminopropanverbindung der Formel I zur Herstellung eines analgetischen Medikaments.

Vorzugsweise werden Verbindungen der Formel I zur Schmerzbehandlung eingesetzt.

Die erfindungsgemäßen Analgetika enthalten neben mindestens einer 1-Phenyl-3-dimethylamino-propanverbindung der Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen derselben hängt davon ab, ob das Arzneimittel oral, intravenös, intraperitoneal, intradermal, intramuskulär, intranasal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der Formel I verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 50 bis 500 mg/kg wenigstens einer 1-Phenyl-3-dimethylamino-propanverbindung der Formel I appliziert.

### Beispiele

Die Ausbeuten der hergestellten Verbindungen sind nicht optimiert.

Alle Temperaturen sind unkorrigiert.

Soweit nicht anders angegeben, wurde Petrolether mit dem Siedebereich 50 - 70° C benutzt. Die Angabe Ether bedeutet Diethylether.

Als stationäre Phase für die Säulenchromatographie wurde Kieselgel 60 (0.040 - 0.063 mmm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Racemattrennungen wurden auf einer Chiracel OD Säule durchgeführt.

Die Mischungsverhältnisse der Laufmittel für alle chromatographischen Untersuchungen sind in Volumen/Volumen angegeben.

RT bedeutet Raumtemperatur, Schmp. Schmelzpunkt.

### Beispiel 1

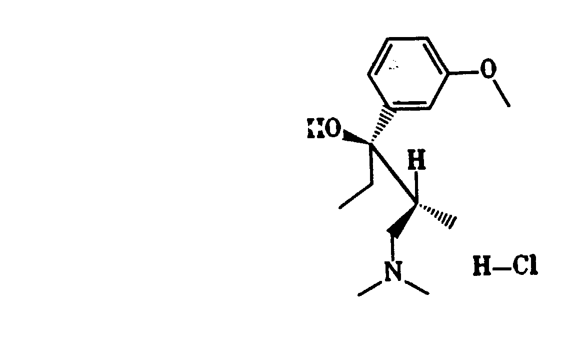

(2RS,3RS)-1-Dimethylamino-3-(3-methoxy-phenyl)-2-methylpentan-3-ol, Hydrochlorid (**1**)

Zu 26,99 g (1,11 mol) Magnesiumspänen in 150 ml trockenem Tetrahydrofuran wurden tropfenweise 207,63 g (1,11 mol) 3-Bromanisol, gelöst in 400 ml trockenem Tetrahydrofuran, so zugegeben, daß das Reaktionsgemisch leicht siedete. Nach vollständiger Zugabe von 3-Bromanisol wurde eine Stunde unter Rückfluß erhitzt und danach auf 5 - 10° C abgekühlt. Bei dieser Temperatur wurden 128,30 g (0,89 mol) 1-Dimethylamino-2-methyl-pentan-3-on, gelöst in 400 ml Tetrahydrofuran, zugegeben. Das Reaktionsgemisch wurde über Nacht stehen gelassen und anschließend erneut auf 5 - 10° C abgekühlt. Durch Zugabe von 300 ml 20 %iger Ammoniumchloridlösung wurde die Grignardlösung zersetzt. Die Reaktionsmischung wurde mit 400 ml Ether verdünnt, die organische Phase abgetrennt und die wäßrige Phase zweimal mit 250 ml Ether extrahiert. Die vereinigten organischen Phasen wurden über Natriumsulfat getrocknet. Nach destillativer Entfernung des Lösungsmittels wurde der Rückstand (212 g) in 3200 ml 2-Butanon aufgenommen und mit 120,60 g (1,11 mol) Trimethylchlorsilan und 20 ml Wasser versetzt. Bei 4 - 5° C kristallisierten 121,5 g (38 % der Theorie) Hydrochlorid (**1**) mit einem Schmelzpunkt von 198 - 199° C. aus.

### Beispiel 2

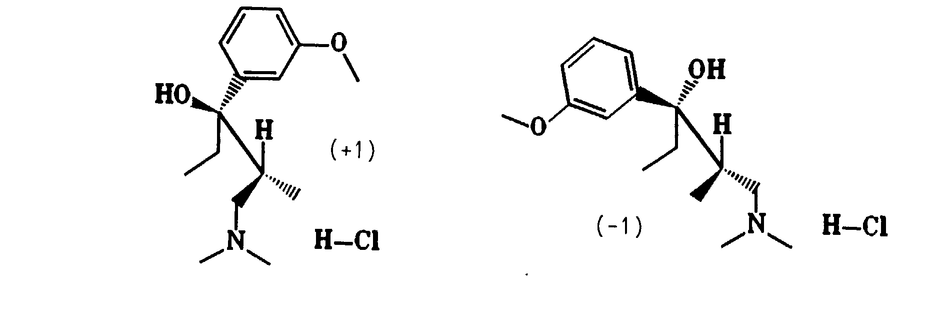

Enantiomere von (**1**):
(-)-(25,35)-1-Dimethylamino-3-(3-methoxy-phenyl)-2-methylpentan-3-ol, Hydrochlorid (**-1**)
und
(+)-(2R,3R)-1-Dimethylamino-3-(3-methoxy-phenyl)-2-methylpentan-3-ol, Hydrochlorid (**+1**)

Aus (**1**) wurde mit Dichlormethan/Natronlauge die Base freigesetzt. Nach dem Trocknen der Lösung wurde Dichlormethan im Vakuum abdestilliert. Das Racemat wurde dann auf der chiralen HPLC-Säule getrennt. Aus den erhaltenen Enantiomeren wurden durch Umsetzung mit Trimethylchlorsilan/Wasser in 2-Butanon die Hydrochloride mit einem Schmelzpunkt von 150 - 151° C hergestellt.
- (**-1**):: Ausbeute: 42 % der Theorie
[α]_{D}^{RT} = -31,8° (c = 0,99; Methanol)
- (**+1**) :: Ausbeute: 41 % der Theorie
[α]_{D}^{RT} = +33,0° (c = 0,96; Methanol)

### Beispiel 3

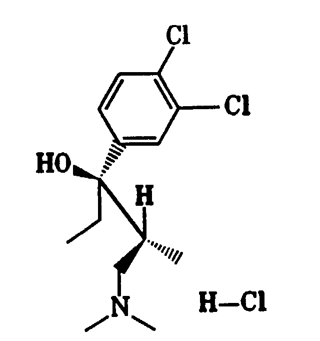

(2RS,3RS)-3-(3,4-Dichlorophenyl)-1-dimethylamino-2-methylpentan-3-ol, Hydrochlorid (**2**)

Analog Beispiel 1 wurden aus 15 g (105 mmol) 1-Dimethylamino-2-methyl-pentan-3-on, 35,5 g (157 mmol) 4-Bromo-1,2-dichlorobenzol und 3,8 g (157 mmol) Magnesiumspänen 39 g Rohgemisch hergestellt. Dieses wurde auf eine 7 x 40 cm Säule, gefüllt mit Kieselgel, gegeben und mit Essigsäureethylester/Methanol 4 : 1 eluiert. Es wurden 14,9 g Base gewonnen, aus der mit Trimethylchlorsilan/Wasser in 2-Butanon/Diisopropylether 11,2 g (31 % der Theorie) Hydrochlorid (**2**) mit einem Schmelzpunkt von 183 - 184° C erhalten wurden.

### Beispiel 4

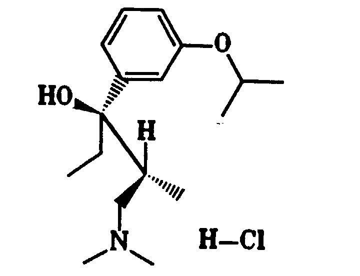

(2RS,3RS)-3-(3-Isopropoxy-phenyl) -1-dimethylamino-2-methyl-pentan-3-ol, Hydrochlorid (**3**)

Entsprechend Beispiel 1 wurden aus 14,3 g (100 mmol) 1-Dimethylamino-2-methyl-pentan-3-on, 20,0 g (157 mmol) 1-Bromo-3-isopropoxy-benzol und 2,79 g (115 mmol) Magnesiumspänen 25 g Rohgemisch hergestellt. Dieses wurde auf eine 7 x 40 cm Säule, gefüllt mit Kieselgel, gegeben und mit Essigsäureethylester/Methanol 15 : 1 eluiert. Es wurden 9,0 g Base gewonnen, aus der mit Trimethylchlorsilan/Wasser in 2-Butanon 8,3 g (26 % der Theorie) Hydrochlorid (**3**) mit einem Schmelzpunkt von 133 - 134° C erhalten wurden.

### Beispiel 5

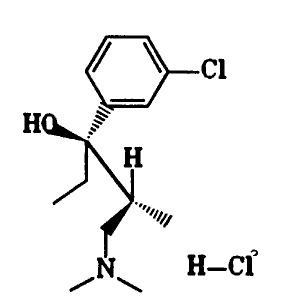

(2RS,3RS)-3-(3-Chloro-phenyl)-1-dimethylamino-2-methylpentan-3-ol, Hydrochlorid (**4**)

Unter den in Beispiel 1 angegebenen Bedingungen wurden aus 38,0 g (270 mmol) 1-Dimethylamino-2-methyl-pentan-3-on, 74,7 g (390 mmol) 1-Bromo-3-chlorobenzol und 9,50 g (390 mmol) Magnesiumspänen 63 g Rohgemisch erhalten. Dieses wurde auf eine 7 x 45 cm Säule, gefüllt mit Kieselgel, gegeben und mit Diisopropylether/Methanol 7 : 1 eluiert. Es wurden 12,8 g Base gewonnen, aus der mit Trimethylchlorsilan/Wasser in 2-Butanon/Ether 10,8 g (14 % der Theorie) Hydrochlorid (**4**) mit einem Schmelzpunkt von 160 - 162° C erhalten wurden.

### Beispiel 6

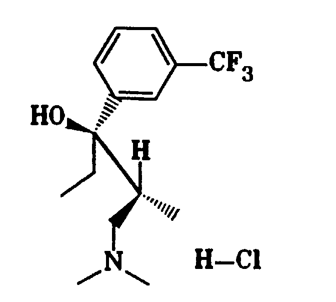

(2RS,3RS)-1-Dimethylamino-2-methyl-3-(3-trifluoromethylphenyl)-pentan-3-ol, Hydrochlorid (**5**)

Unter den in Beispiel 1 angegebenen Bedingungen wurden aus 14,3 g (100 mmol) 1-Dimethylamino-2-methyl-pentan-3-on, 29,3 g (130 mmol) 1-Bromo-3-trifluoromethyl-benzol und 3,2 g (130 mmol) Magnesiumspänen 21,2 g Rohgemisch erhalten. Dieses wurde auf eine 6 x 40 cm Säule, gefüllt mit Kieselgel, gegeben und mit Diisopropylether/Methanol 10 : 1 eluiert. Es wurden 9,1 g Base gewonnen, aus der mit Trimethylchlorsilan/Wasser in 2-Butanon 7,8 g (18,5 % der Theorie) Hydrochlorid (**5**) mit einem Schmelzpunkt von 189 - 190° C erhalten wurden.

### Beispiel 7

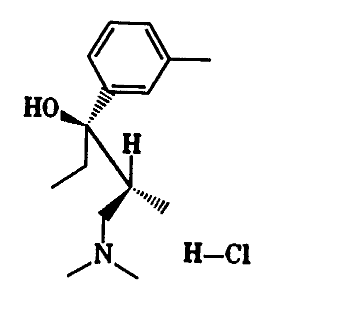

(2RS,3RS)-1-Dimethylamino-2-methyl-3-(m-tolyl)-pentan-3-ol, Hydrochlorid (**6**)

Gemäß Beispiel 1 wurden aus 47,3 g (330 mmol) 1-Dimethylamino-2-methyl-pentan-3-on, 64,6 g (400 mmol) 3-Brom-toluol und 9,72 g (400 mmol) Magnesiumspänen 75 g Rohgemisch erhalten. Dieses wurde auf eine 7 x 50 cm Säule, gefüllt mit Kieselgel, gegeben und mit Diisopropylether/Methanol 7 : 1 eluiert. Es wurden 24,3 g Base gewonnen, aus der mit Trimethylchlorsilan/Wasser in 2-Butanon 21,5 g (24 % der Theorie) Hydrochlorid (**6**) mit einem Schmelzpunkt von 154 - 155° C erhalten wurden.

### Beispiel 8

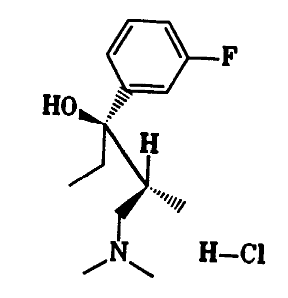

(2RS,3RS)-1-Dimethylamino-3-(3-fluoro-phenyl)-2-methylpentan-3-ol, Hydrochlorid (**7**)

Unter den in Beispiel 1 angegebenen Bedingungen wurden aus 54,0 g (380 mmol) 1-Dimethylamino-2-methyl-pentan-3-on, 82,5 g (470 mmol) 1-Bromo-3-fluoro-benzol und 9,23 g (470 mmol) Magnesiumspänen 70 g Rohgemisch erhalten. Dieses wurde auf eine 7 x 50 cm Säule, gefüllt mit Kieselgel, gegeben und mit Essigsäureethylester/Methanol 1 : 1 eluiert. Es wurden 13,0 g Base gewonnen, aus der mit Trimethylchlorsilan/Wasser in 2-Butanon 11,2 g (11,5 % der Theorie) Hydrochlorid (**7**) mit einem Schmelzpunkt von 145 - 146° C erhalten wurden.

### Beispiel 9

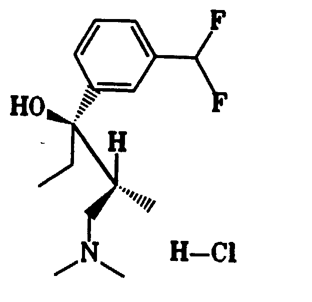

(2RS,3RS)-3-(3-Difluoromethyl-phenyl)-1-dimethylamino-2-methyl-pentan-3-ol, Hydrochlorid (**8**)

7,0 g (34 mmol) 1-Bromo-3-difluoromethyl-benzol, hergestellt aus 3-Bromobenzaldehyd und Diethylaminoschwefeltrifluorid in Dichlormethan gemäß Org. React. 35, 513 (1988), wurden in 110 ml trockenem Tetrahydrofuran gelöst und auf -75° C gekühlt. Nach Zugabe von 21,12 ml (34 mmol) 1,6 molarer n-Butyllithiumlösung in Hexan wurde eine Stunde bei -75° C gerührt. Anschließend wurden 4,8 g (34 mmol) l-Dimethylamino-2-methyl-pentan-3-on, gelöst in 15 ml trockenem Tetrahydrofuran, zugetropft. Innerhalb von 2,5 Stunden wurde das Reaktionsgemisch auf Raumtemperatur erwärmt.

Zur Aufarbeitung wurden unter Eisbadkühlung 65 ml 5 %ige Salzsäure zugetropft, so daß die Innentemperatur 15° C nicht überstieg. Nach Phasentrennung wurde die organische Phase mit 40 ml 5 %iger Salzsäure extrahiert. Die vereinigten wäßrigen Phasen wurden zweimal mit 50 ml Ether gewaschen. Zur Freisetzung der Base wurde mit konzentrierter Natronlauge versetzt und mit Dichlormethan extrahiert. Auf diese Weise wurden 7,8 g Rohprodukt erhalten, welches auf eine 7 x 40 cm Säule, gefüllt mit Kieselgel, gegeben wurde. Die Elution mit Essigsäureethylester/Methanol = 1 : 1 ergab 4,89 g Base, aus der mit Trimethylchlorsilan/Wasser in 2-Butanon 4,6 g (44 % der Theorie) Hydrochlorid (**8**) mit einem Schmelzpunkt von 194 - 195° C erhalten wurden.

### Beispiel 10

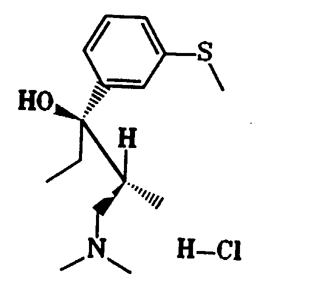

(2RS,3RS)-1-Dimethylamino-2-methyl-3-(3-methylsulfanylphenyl)-pentan-3-ol, Hydrochlorid (**9**)

Unter den in Beispiel 1 angegebenen Bedingungen wurden aus 17,6 g (123 mmol) 1-Dimethylamino-2-methyl-pentan-3-on, 25,0 g (123 mmol) 1-Bromo-3-methylsulfanyl-benzol und 3,0 g (123 mmol) Magnesiumspänen 38 g Rohgemisch erhalten. Dieses wurde auf eine 7 x 40 cm Säule, gefüllt mit Kieselgel, gegeben und mit Essigsäureethylester/Methanol 10 : 1 eluiert. Es wurden 8,35 g Base gewonnen, aus der mit Trimethylchlorsilan/Wasser in 2-Butanon 7,2 g (19 % der Theorie) Hydrochlorid (**9**) mit einem Schmelzpunkt von 159 - 160° C erhalten wurden.

### Beispiel 11

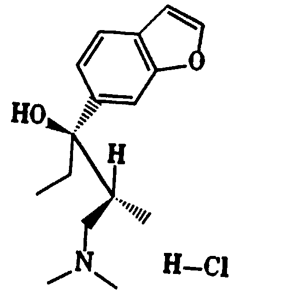

(2RS,3RS)-3-Benzofuran-6-yl-1-dimethylamino-2-methyl-pentan-3-ol, Hydrochlorid (**10**)

Zu 2,12 g (87 mmol) Magnesiumspänen in 30 ml trockenem Ether wurden 3,45 g (18 mmol) 6-Bromo-benzofuran (hergestellt nach EP 355 827) und 6 ml 1,2-Dibromo-ethan, gelöst in 60 ml trockenem Ether, innerhalb von 1,5 Stunden getropft und nach Zugabe 30 Minuten unter Rückfluß erhitzt. Danach wurden unter Eisbadkühlung bei 5 - 10° C Innentemperatur 2,5 g (18 mmol) 1-Dimethylamino-2-methyl-pentan-3-on, gelöst in 7,5 ml Ether, zugetropft. Das Reaktionsgemisch wurde 12 Stunden bei Raumtemperatur stehen gelassen, dann erneut auf 5 - 10° C abgekühlt und mit 35 ml 20 %iger, wäßriger Ammoniumchloridlösung versetzt. Nach Phasentrennung wurde die wäßrige Phase zweimal mit 50 ml Ether extrahiert. Die vereinigten organischen Phasen wurden über Natriumsulfat getrocknet. Nach destillativer Entfernung des Lösungsmittels wurde der Rückstand (3,9 g) auf eine 5 x 16 cm Säule, gefüllt mit Kieselgel, gegeben. Durch Elution mit Diisopropylether/Methanol 7 : 1 wurden 0,95 g Base gewonnen, aus der mit Trimethylchlorsilan/Wasser in Essigsäureethylester/2-Butanon 0,82 g (15,5 % der Theorie) Hydrochlorid (**10**) mit einem Schmelzpunkt von 162° C erhalten wurden.

### Beispiel 12

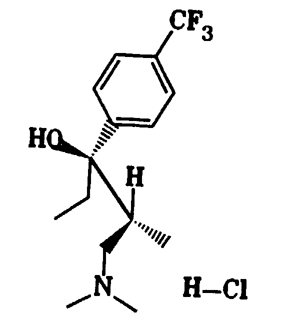

(2RS,3RS)-1-Dimethylamino-2-methyl-3-(4-trifluoromethylphenyl)-pentan-3-ol, Hydrochlorid (**11**)

Gemäß Beispiel 1 wurden aus 20 g (140 mmol) 1-Dimethylamino-2-methyl-pentan-3-on, 31,5 g (140 mmol) 1-Bromo-4-trifluoromethyl-benzol, 16,5 g (680 mmol) Magnesiumspänen und 47 ml 1,2-Dibromo-ethan 44 g Rohgemisch erhalten. Dieses wurde auf eine 7 x 50 cm Säule, gefüllt mit Kieselgel, gegeben und mit Essigsäureethylester/Methanol 5 : 1 eluiert. Es wurden 16,4 g Base gewonnen, aus der mit Trimethylchlorsilan/Wasser in 2-Butanon 12,3 g (27 % der Theorie) Hydrochlorid (**11**) mit einem Schmelzpunkt von 170 - 171° C erhalten wurden.

### Beispiel 13

(3RS)-1-Dimethylamino-3-(3-methoxy-phenyl)-hexan-3-ol, Hydrochlorid (**12**)

Gemäß Beispiel 1 wurden aus 10 g (70 mmol) 1-Dimethylamino-hexan-3-on, 18,7 g (100 mmol) 1-Bromo-3-methoxy-benzol und 2,3 g (100 mmol) Magnesiumspänen 18,5 g Rohgemisch erhalten. Dieses wurde auf eine 6 x 50 cm Säule, gefüllt mit Kieselgel, gegeben und mit Essigsäureethylester/Methanol 1 : 1 eluiert. Es wurden 6,84 g Base gewonnen, aus der mit Trimethylchlorsilan/Wasser in 2-Butanon 6,15 g (32 % der Theorie) Hydrochlorid (**12**) mit einem Schmelzpunkt von 179 - 180° C erhalten wurden.

### Beispiel 14

(3RS)-1-Dimethylamino-3-(3-methoxy-phenyl)-heptan-3-ol, Hydrochlorid (**13**)

Gemäß Beispiel 1 wurden aus 10 g (64 mmol) 1-Dimethylamino-heptan-3-on, 15,9 g (157 mmol) 1-Bromo-3-methoxy-benzol und 2,06 g (85 mmol) Magnesiumspänen 17,3 g Rohgemisch erhalten. Dieses wurde auf eine 6 x 40 cm Säule, gefüllt mit Kieselgel, gegeben und mit Essigsäureethylester eluiert. Es wurden 5,4 g Base gewonnen, aus der mit Trimethylchlorsilan/Wasser in 2-Butanon 4,1 g (21 % der Theorie) Hydrochlorid (**13**) mit einem Schmelzpunkt von 150° C erhalten wurden.

### Beispiel 15

(3RS)-1-Dimethylamino-3-(3-methoxy-phenyl)-4,4-dimethylpentan-3-ol, Hydrochlorid (**14**)

Gemäß Beispiel 1 wurden aus 18,6 g (118 mmol) 1-Dimethylamino-4,4-dimethyl-pentan-3-on, 28,4 g (152 mmol) 1-Bromo3-methoxy-benzol und 3,7 g (152 mmol) Magnesiumspänen 37 g Rohgemisch erhalten. Dieses wurde auf eine 7 x 40 cm Säule, gefüllt mit Kieselgel, gegeben und mit Essigsäureethylester/Methanol 5 : 1 eluiert. Es wurden 2,2 g Base gewonnen, aus der mit Trimethylchlorsilan/Wasser in 2-Butanon 1,8 g (5 % der Theorie) Hydrochlorid (**14**) mit einem Schmelzpunkt von 213° C erhalten wurden.

### Beispiel 16

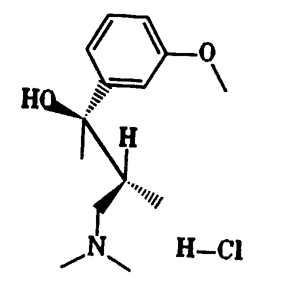

(2RS,3RS)-4-Dimethylamino-2-(3-methoxy-phenyl)-3-methylbutan-2-ol, Hydrochlorid (**15**)

Gemäß Beispiel 1 wurden aus 5,3 g (41 mmol) 4-Dimethylamino-3-methyl-butan-2-on, 23,0 g (123 mmol) 1-Bromo-3-methoxy-benzol und 3,0 g (123 mmol) Magnesiumspänen 21 g Rohgemisch erhalten. Dieses wurde auf eine 4,5 x 27 cm Säule, gefüllt mit Kieselgel, gegeben und mit Essigsäureethylester/Methanol 4 : 1 eluiert. Es wurden 4,0 g Base gewonnen, aus der mit Trimethylchlorsilan/Wasser in 2-Butanon 3,6 g (32 % der Theorie) Hydrochlorid (**15**) mit einem Schmelzpunkt von 124° C erhalten wurden.

### Beispiel 17

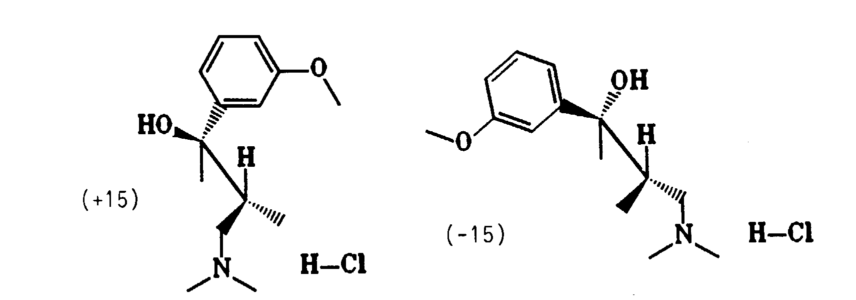

Enantiomere von (**15**):
(-)-(2S,3S)-4-Dimethylamino-2-(3-methoxy-phenyl)-3-methylbutan-2-ol, Hydrochlorid (**-15**)
und
(+)-(2R,3R)-4-Dimethylamino-2-(3-methoxy-phenyl)-3-methylbutan-2-ol, Hydrochlorid (**+15**)

Aus dem nach Beispiel 16 hergestellten Hydrochlorid (**15**) wurde mit Dichlormethan/Natronlauge die Base freigesetzt. Nach Trocknung und destillativer Entfernung von Dichlormethan wurde das Racemat auf einer chiralen HPLC-Säule in die Enantiomeren getrennt. Aus diesen wurden mit Trimethylchlorsilan/Wasser in 2-Butanon die Hydrochloride erhalten.
- (**-15**) :: Ausbeute: 41 % der Theorie
Schmp: 117 - 118 °C
[α]_{D}^{RT} = -38,6° (c = 1,05; Methanol)
- (**+15**):: Ausbeute: 41 % der Theorie
Schmp: 118 - 119° C
[α]_{D}^{RT} = +41,0° (c = 1,01; Methanol)

### Beispiel 18

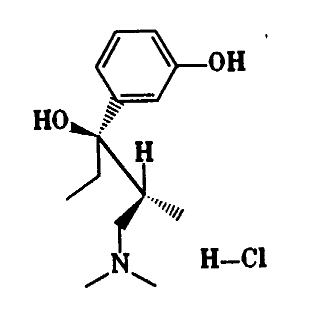

(2RS,3RS)-3-(3-Dimethylamino-1-ethyl-1-hydroxy-2-methylpropyl)-phenol, Hydrochlorid (**16**)

Aus der nach Beipiel 1 erhaltenen Verbindung (1) wurde mit Dichlormethan/Natronlauge die Base freigesetzt und nach Trocknung der Lösung Dichlormethan destillativ entfernt. 4,3 g (17 mmol) Base wurden in 25 ml trocknenem Toluol gelöst und langsam zu 71 ml (85 mmol) 1,2-molarer toluolischer Diisobutylaluminiumhydrid-Lösung getropft. Nach vollständiger Zugabe wurde 8 Stunden unter Rückfluß erhitzt und anschließend auf Raumtemperatur abgekühlt.Das Reaktionsgemisch wurde mit 25 ml Toluol verdünnt. Unter Eisbadkühlung wurden 9,4 ml Ethanol und anschließend 9,4 ml Wasser zugetropft. Nach einstündigem Rühren unter Eisbadkühlung wurde das Reaktionsgemisch durch Filterung von Aluminiumsalzen befreit, wobei der Rückstand dreimal mit je 50 ml Toluol gewaschen wurde. Danach wurden die vereinigten organischen Phasen getrocknet und Toluol destillativ entfernt. Aus der Base wurden in Aceton mit wäßriger Salzsäurelösung in Aceton 3,95 g (85 % der Theorie) Hydrochlorid (**16**) mit einem Schmelzpunkt von 213 - 214° C erhalten.

### Beispiel 19

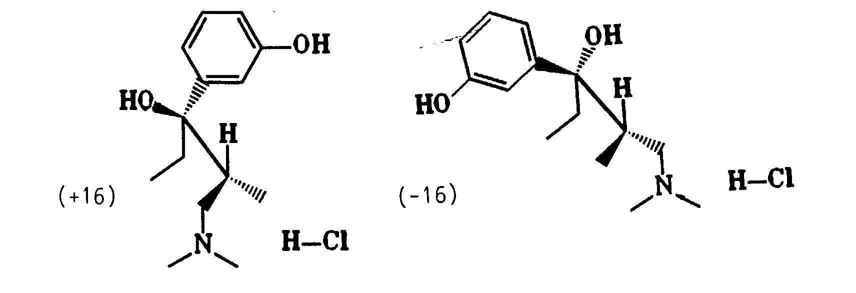

Enantiomere von (**16**):
(-) (25,35)-3-(3-Dimethylamino-1-ethyl-1-hydroxy-2-methylpropyl)-phenol, Hydrochlorid (**-16**)
und
(+) (2R,3R)-3-(3-Dimethylamino-1-ethyl-1-hydroxy-2-methylpropyl)-phenol, Hydrochlorid (**+16**)

Die Enantiomeren (**-16**) und (**+16**) wurden unter den in Beispiel 2 angegebenen Bedingungen hergestellt.
- (**-16**):: Ausbeute: 85 % der Theorie
Schmp: 208 - 209° C
[α]_{D}^{RT} = -34,6° (c = 0,98; Methanol)
- (**+16**):: Ausbeute: 85 % der Theorie
Schmp: 206 - 207° C
[α]_{D}^{RT} = +34,4 ° (c = 1,06; Methanol)

### Beispiel 20

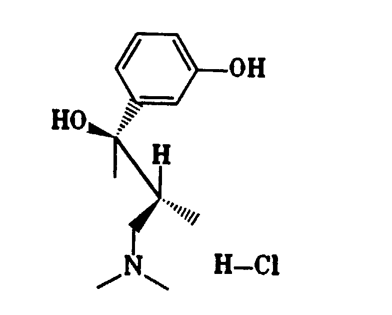

(1RS,2RS)-3-(3-Dimethylamino-l-hydroxy-1,2-dimethyl-propyl)-phenol, Hydrochlorid (**17**)

Unter den in Beispiel 18 angegebenen Bedingungen wurde Verbindung (**17**), ausgehend von der nach Beispiel 16 erhaltenen Methoxyverbindung (**15**) hergestellt.
Ausbeute: 85 % der Theorie
Schmp: 232° C

### Beispiel 21

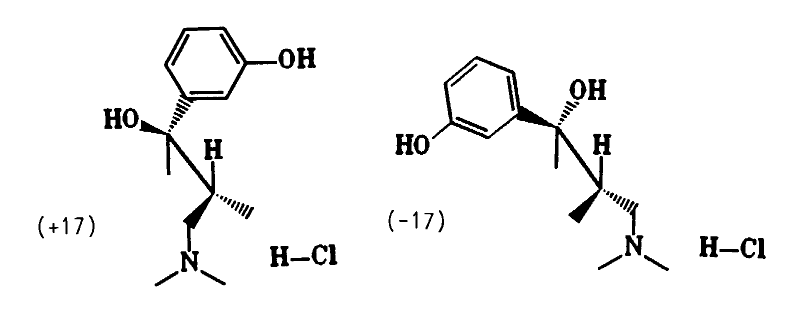

Enantiomere von (**17**):
(-)-(1S,2S)-3-(3-Dimethylamino-1-hydroxy-1,2-dimethyl-propyl)-phenol, Hydrochlorid (**-17**)
und
(+)-(1R,2R)-3-(3-Dimethylamino-1-hydroxy-1,2-dimethyl-propyl)-phenol, Hydrochlorid (**+17**)

Die Enantiomeren (**-17**) und (**+17**) wurden unter den in Beispiel 2 angegebenen Bedingungen hergestellt.
- (**-17**):: Ausbeute: 82 % der Theorie
Schmp: 204 - 205° C
[α]_{D}^{RT} = -42,0° (c = 0,94; Methanol)
- (**+17**):: Ausbeute: 83 % der Theorie
Schmp: 204 - 205 °C
[α]_{D}^{RT} = +41,2° (c = 1,01; Methanol)

### Beispiel 22

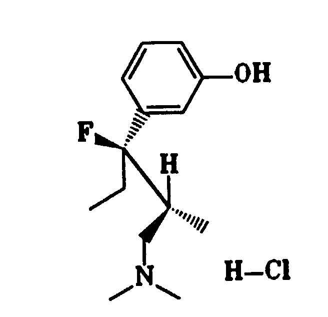

(+)-(1R,2R)-3-(3-Dimethylamino-1-ethyl-1-fluoro-2-methylpropyl)-phenol, Hydrochlorid (**+18**)

### 1. Stufe

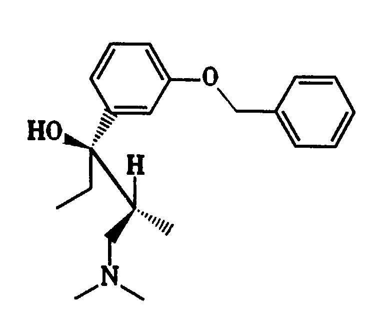

(+)-(1R,2R)-3-(3-Benzyloxy-phenyl)-1-dimethylamino-2-methyl-pentan-3-ol (**+19**)

Aus dem gemäß Beispiel 19 erhaltenen Enantiomeren (**+16**) wurde mit Dichlormethan/Natronlauge die Base freigesetzt und nach Trocknung der Lösung Dichlormethan destillativ entfernt. 5,3 g (22 mmol) Base wurden in 27 ml trockenem Dimethylformamid gelöst und mit 1,2 g 50 %igem Natriumhydrid in mehreren Portionen versetzt. Nach Zugabe von 2,8 ml (24 mmol) Benzoylchlorid wurde drei Stunden auf 70° C erhitzt. Anschließend wurde auf Raumtemperatur abgekühlt und das Reaktionsgemisch auf Eiswasser gegossen. Es wurde dreimal mit je 70 ml Ether extrahiert. Nach Trocknung der vereinigten organischen Phasen über Natriumsulfat wurde das Lösungsmittel abdestilliert und der Rückstand auf eine 4,5 x 30 cm Säule, gefüllt mit Kieselgel, gegeben. Durch Elution mit Diisopropylether/Methanol wurden 6,8 g (92 % der Theorie) Base (**+19**) als hellgelbes, hochviskoses Öl erhalten.

### 2. Stufe

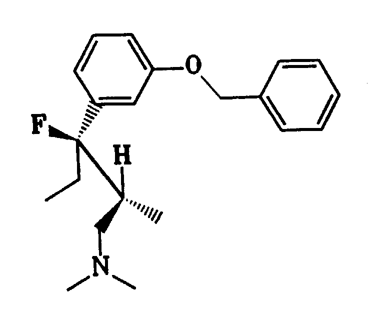

(+)-(2R,3R)-[3-(3-Benzyloxy-phenyl)-3-fluoro-2-methyl-pentyl]-dimethylamin (**+20**)

Zu einer Lösung von 3,7 g (23 mmol) Diethylamino-schwefeltrifluorid in 30 ml trockenem Dichlormethan wurden bei -20° C 6,8 g (21 mmol) (**+19**), gelöst in 80 ml Dichlormethan, getropft. Nach vollständiger Zugabe wurde 30 Minuten bei dieser Temperatur gerührt und anschließend auf Raumtemperatur erwärmt. Nach weiterem einstündigen Rühren bei Raumtemperatur wurde auf 0 - 5° C gekühlt und mit 50 ml Wasser hydrolysiert. Nach Phasentrennung wurde die wäßrige Phase zweimal mit 50 ml Dichlormethan extrahiert. Die vereinigten organischen Phasen wurden getrocknet und durch Destillation im Vakuum vom Lösungsmittel befreit. Das erhaltene Rohgemisch (8,04 g) wurde auf eine 6 x 50 cm Säule, gefüllt mit Kieselgel, gegeben und mit Essigsäureethylester/Methanol = 1 : 1 eluiert. Es wurden 3,04 g (40 % der Theorie) Base (**+20**) als hellgelbes viskoses Öl erhalten.

### 3.Stufe:

(+)-(1R,2R)-3-(3-Dimethylamino-1-ethyl-1-fluoro-2-methylpropyl)-phenol, Hydrochlorid (**+18**)

3.0 g (91 mmol) (**+20**) wurden in 15 ml trockenem Methanol gelöst und in einer Hydrierapparatur mit 0,44 g Palladium auf Aktivkohle (10 % Pd) versetzt. Nach drei Stunden Rühren bei Raumtemperatur waren 215 ml Wasserstoff verbraucht. Der Katalysator wurde durch Filtration und Methanol durch Destillation entfernt. Es wurden 2,22 g Base gewonnen, aus der mit Trimethylchlorsilan/Wasser in 2-Butanon 2,0 g (79 % der Theorie) Hydrochlorid (**+18**) erhalten wurden.
Schmp: 174 -176 °C
[α]_{D}^{RT} = + 29,5° (c = 1,08; Methanol)

### Beispiel 23

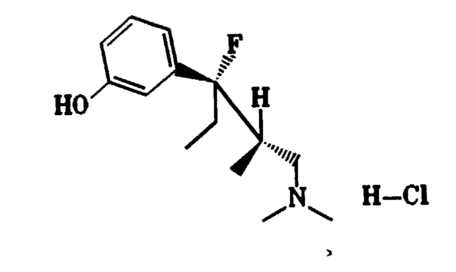

(-)-(1S,2S)-3-(3-Dimethylamino-1-ethyl-1-fluoro-2-methylpropyl)-phenol, Hydrochlorid (**-18)**

Ausgehend von dem gemäß Beispiel 19 erhaltenen Enantiomeren (**-16**) wurde unter den in Beispiel 22 angegebenen Bedingungen das Enantiomere (-18) in einer Ausbeute von 29 % der Theorie erhalten.
Schmp: 170 - 172° C
[α]_{D}^{RT} = - 28,4° (c = 1,03; Methanol)

### Beispiel 24

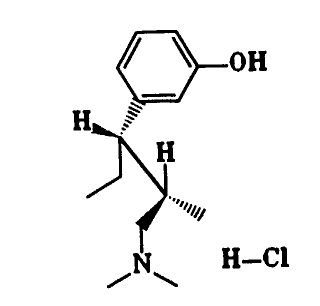

(+)-(1R,2R)-3-(3-Dimethylamino-1-ethyl-2-methyl-propyl)phenol, Hydrochlorid (**+21**)

### 1. Stufe

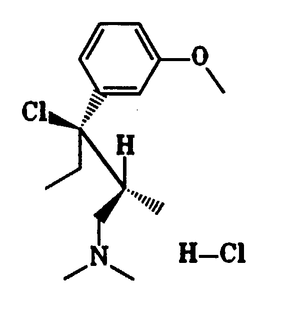

(+)-(2R,3R)-[3-Chloro-3-(-3-methoxy-phenyl)-2-methyl-pentyl]-dimethylamin, Hydrochlorid (**+22**)

10g (35 mmol) (**+1**), hergestellt nach Beispiel 2, wurden bei Raumtemperatur mit 10 ml Thionylchlorid versetzt. Zur Entfernung von überschüssigem Thionylchlorid wurde anschließend zwei Stunden Stickstoff über das Reaktionsgemisch geleitet. Nach erneuter Zugabe von 10 ml Thionylchlorid wurde das Reaktionsgemisch 12 Stunden stehen gelassen, bevor innerhalb von 2,5 Stunden überschüssiges Thionylchlorid mit Hilfe eines Stickstoffstromes erneut entfernt wurde. Nach Trocknung wurde der Rückstand in 10 ml eiskaltem 2-Butanon gelöst und unter Rühren mit 200 ml Ether und anschließend mit 140 ml Diisopropylether versetzt. Die überstehende Lösungsmittelphase wurde abdekantiert und das zurückbleibende Öl erneut in 10 ml 2-Butanon aufgenommen. Nach Zugabe von Impfkristallen wurden 300 ml Diisopropylether innerhalb von drei Stunden unter kräftigem Rühren zugetropft, wobei das Hydrochlorid auskristallisierte. Es wurden 9,8 g (91 % der Theorie) (**22**) erhalten.
Schmp: 120° C (Zersetzung)
[α]_{D}^{RT} = +24,7° (c = 1,01; Methanol)

### 2. Stufe

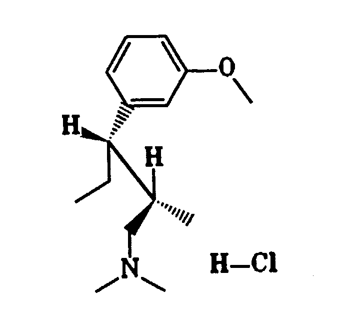

(+)-(2R,3R)-[3-(3-Methoxy-phenyl)-2-methyl-pentyl]-dimethylamin, Hydrochlorid (**+23**)

46 g getrocknetes Zinkchlorid wurden in 580 ml trockenem Ether gelöst und anschließend zu einer Aufschlämmung von 31 g Natriumborhydrid in 1800 ml Ether getropft. Nach 12stündigem Rühren wurden von der erhaltenen Zinkborhydridsuspension 500 ml abdekantiert und tropfenweise zu 9,8 g (32 mmol) (**+22**) in 200 ml trockenem Ether gegeben. Das Reaktionsgemisch wurde 72 Stunden bei Raumtemperatur gerührt und anschließend unter Eisbadkühlung tropfenweise mit 40 ml einer gesättigten Ammoniumchloridlösung versetzt. Nach Phasentrennung wurde die Etherphase zweimal mit gesättigter Kochsalzlösung gewaschen und nach dem Trocknen über Natriumsulfat das Lösungsmittel im Vakuum abdestilliert. Es wurden 7,3 g eines Amin-Boran-Komplexes erhalten, der zur Isolierung der freien Base in 100 ml trockenem Methanol gelöst wurde. Nach Zugabe von 7,5 g Triphenylphosphin wurde 18 Stunden unter Rückfluß erhitzt. Nach destillativer Entfernung des Lösungsmittels wurde der Rückstand mit 100 ml 5 %iger Salzsäure versetzt, wobei anschließend die Salzsäurephase noch zweimal mit 50 ml Ether gewaschen wurde. Danach wurde die Salzsäurephase mit konzentrierter Natronlauge unter Eisbadkühlung alkalisiert und zweimal mit 50 ml Dichlormethan ausgeschüttelt. Nach dem Trocknen der vereinigten organischen Phasen über Natriumsulfat wurde das Lösungsmittel im Vakuum abdestilliert und der verbleibende Rückstand (5,2 g) in 2-Butanon aufgenommen. Nach der Zugabe von Trimethylchlorsilan/Wasser kristallisierten 4,3 g (50 % der Theorie) Hydrochlorid (**+23**) aus.
Schmp: 163 - 164°C
[α]_{D}^{RT} = +25,2° (c = 0,95; Methanol)

### 3.Stufe

(+)-(1R,2R)-3-(3-Dimethylamino-1-ethyl-2-methyl-propyl)phenol, Hydrochlorid (**+21**)

4,3 g (15 mmol) (**+23**) aus Stufe 2 wurden mit 100 ml konzentrierter Bromwasserstoffsäure versetzt. Anschließend wurde zwei Stunden unter Rückfluß erhitzt. Nach dem Abkühlen auf Raumtemperatur wurde das Reaktionsgemisch im Wasserstrahlvakuum eingeengt. Der Rückstand wurde bis zur alkalischen Reaktion mit konzentrierter Natriumhydrogencarbonatlösung versetzt. Nach zweimaliger Extraktion mit je 50 ml Dichlormethan wurden die vereinigten organischen Phasen über Natriumsulfat getrocknet. Anschließend wurde Dichlormethan im Vakuum abdestilliert und der Rückstand (4 g) in 2-Butanon aufgenommen. Nach der Zugabe von Trimethylchlorsilan/Wasser kristallisierten 3,8 g (98 % der Theorie) Hydrochlorid (**+21**) aus.
Schmp: 194 -196°C
[α]_{D}^{RT} = + 24,5° (c = 1,10; Methanol)

### Beispiel 25

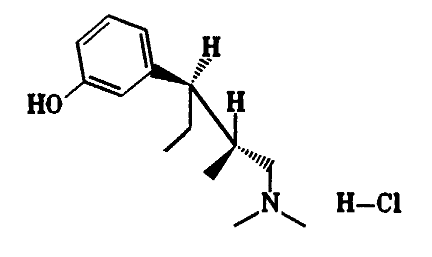

(-)-(1S,2S)-3-(3-Dimethylamino-1-ethyl-2-methyl-propyl)phenol, Hydrochlorid (**-21**)

Ausgehend von (**-1**), hergestellt nach Beispiel 2, wurde das Enantiomere (**-21**) unter den in Beispiel 24 angegebenen Bedingungen in 45 %iger Ausbeute erhalten.
Schmp: 168 -170 °C
[α]_{D}^{RT} = - 27,5° (c = 0,97; Methanol)

### Beispiel 26

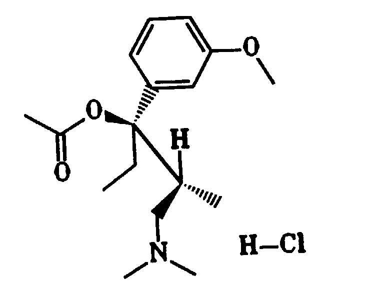

(+)-(1R,2R)-Essigsäure-3-dimethylamino-1-ethyl-1-(3-methoxy-phenyl)-2-methyl-propylester, Hydrochlorid (**+24**)

Aus dem Enantiomeren (**+1**), hergestellt nach Beispiel 2, wurde mit Dichlormethan/Natronlauge die Base freigesetzt. Nach dem Trocknen der Lösung wurde Dichlormethan destillativ entfernt. Zu 10 g (35 mmol) der erhaltenen Base, die in 150 ml trockenem Dichlormethan aufgenommen wurde, wurden unter Einbadkühlung 3,0 g (39 mmol) Acetylchlorid getropft. Nach vollständiger Acetylchloridzugabe wurde das Reaktionsgemisch auf Raumtemperatur erwärmt und nach zweistündigem Rührem mit 100 ml gesättigter Natriumhydrogencarbonatlösung versetzt. Die organische Phase wurde von der wäßrigen Phase abgetrennt und die wäßrige Phase zweimal mit 50 ml Dichlormethan extrahiert. Die organischen Phasen wurden vereinigt und über Natriumsulfat getrocknet. Nach destillativer Entfernung des Lösungsmittels wurden 13,4 g Rohgemisch gewonnen, aus dem mit Trimethylchlorsilan/Wasser in 2-Butanon/Ethylacetat 10,7 g (93 % der Theorie) Hydrochlorid (**+24**) erhalten wurden.
Schmp: 153° C
[α]_{D}^{RT} = + 17,3° (c = 1,04; Methanol)

### Beispiel 27

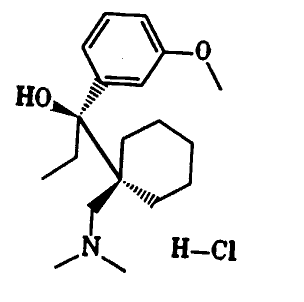

(1RS)-1-(1-Dimethylaminomethyl-cyclohexyl)-1-(3-methoxyphenyl)-propan-1-ol, Hydrochlorid (**25**)

### 1. Stufe

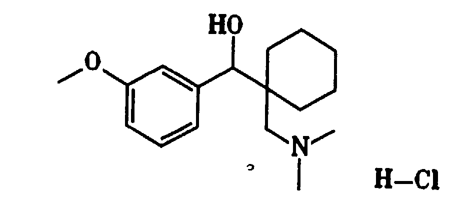

(1RS)-(1-Dimethylaminomethyl-cyclohexyl)-(3-methoxy-phenyl)-methanol, Hydrochlorid (**26**)

Unter den in Beispiel 1 angegebenen Bedingungen wurden aus 25 g (150 mmol) 1-Dimethylaminomethyl-cyclohexan-carbaldehyd, 32,9 g (180 mmol) 1-Bromo-3-methoxy-benzol und 4,3 g (180 mmol) Magnesiumspänen 44 g Rohgemisch erhalten. Dieses wurde auf eine 7 x 40 cm Säule, gefüllt mit Kieselgel, gegeben und mit Diisopropylether/Methanol 4 : 1 eluiert. Es wurden 38 g Base gewonnen, aus der mit Trimethylchlorsilan/Wasser in 2-Butanon 40 g (85 % der Theorie) Hydrochlorid (26) mit einem Schmelzpunkt von 235° C erhalten wurden.

### 2. Stufe

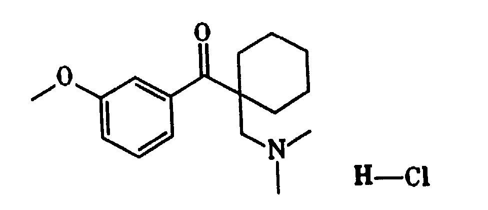

(1RS)-(1-Dimethylaminomethyl-cyclohexyl)-(3-methoxy-phenyl)-methanon, Hydrochlorid (**27**)

Aus (**26**) wurde mit Dichlormethan/Natronlauge die Base freigesetzt und nach dem Trocknen der Lösung Dichlormethan destillativ entfernt. 8,3 g (30 mmol) Base wurden in 30 ml n-Hexan gelöst und zu einer Suspension, bestehend aus 95 g auf neutralem Aluminiumoxid absorbiertem Pyridiniumchlorochromat (hergestellt gemäß Synthesis 1980, 223) getropft. Nach 72stündigem Rühren bei Raumtemperatur wurde das Reaktionsgemisch mit 120 ml Dichlormethan versetzt, weitere 2 Stunden gerührt und anschließend über 30 g Aluminiumoxid filtriert. Der Filterrückstand wurde dreimal mit je 50 ml Dichlormethan und Ether dekantierend gewaschen. Die mit dem Filtrat vereinigten organischen Phasen wurden destillativ von Lösungsmittel befreit, der erhaltene Rückstand in 60 ml 2-normaler Natronlauge aufgenommen und viermal mit je 20 mg Ethylacetat extrahiert. Nach dem Trocknen der vereinigten organischen Phasen wurde das Lösungsmittel destillativ entfernt. Es wurden 4,8 g Rohgemisch gewonnen, die auf eine 6 x 30 cm-Säule, gefüllt mit Kieselgel, gegeben und zunächst mit Ethylacetat, dann mit Ethylacetat/Methanol 9 : 1 und schließlich mit Ethylacetat/Methanol 4 : 1 eluiert wurden. Es wurden 3,8 g Base gewonnen, aus der mit Trimethylchlorsilan/Wasser in 2-Butanon 3,1 g (33 % der Theorie) Hydrochlorid (**27**) mit einem Schmelzpunkt von 174° C erhalten wurden.

### 3. Stufe

(1RS)-1-(1-Dimethylaminomethyl-cyclohexyl)-1-(3-methoxyphenyl)-propan-1-ol, Hydrochlorid (**25**)

Unter den in Beispiel 1 angegebenen Bedingungen wurden aus 2,8 g (10 mmol) (**27**) in Form der Base, 1,4 g (13 mmol) Bromethan und 0,32 g (13 mmol) Magnesiumspänen unter Verwendung von Ether als Lösungsmittel 3,0 g Rohgemisch erhalten. Dieses wurde auf eine 3 x 20 cm Säule, gefüllt mit Kieselgel, gegeben und mit Diisopropylether/Methanol 19 : 1 eluiert. Es wurden 2,1 g Base gewonnen, aus der mit Trimethylchlorsilan/Wasser in 2-Butanon/Ethylacetat 1,9 g (55 % der Theorie) Hydrochlorid (**25**) mit einem Schmelzpunkt von 230° C erhalten wurden.

### Beispiel 28

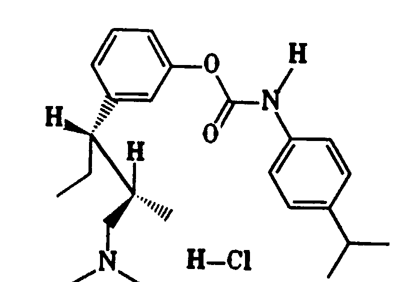

(-)-(2R,3S)-(3[3-(p-Isopropyl-phenyl-carbamoyl)-oxy-phenyl]-2-methyl-pentyl)-dimethylamin, Hydrochlorid (**-28**)

Aus dem Enantiomeren (**+21**), hergestellt nach Beispiel 24, wurde mit Dichlormethan/Natronlauge die Base freigesetzt und nach dem Trocknen der Lösung Dichlormethan destillativ entfernt. 2,2 g (10 mmol) der erhaltenen Base wurden in 20 ml trockenem Toluol gelöst und mit 1,8 g (11 mmol) 4-Isopropylphenylisocyanat versetzt. Nach 20stündigem Rühren bei Raumtemperatur wurde Toluol destillativ entfernt. Der Rückstand wurde mit Trimethylchlorsilan/Wasser in n-Propylacetat zu 3,2 g (76 % der Theorie) Hydrochlorid (**-28**) umgesetzt.
Schmp.: 151 - 152° C
[α]^{RT}_{D} = - 5,2° (c = 1,11; Methanol)

### Pharmakologische Untersuchungen

### Writhing-Test an der Maus

Die analgetische Wirksamkeit der erfindungsgemäßen Verbindungen wurde im Phenylchinon-induzierten Writhing-Test, modifiziert nach I.C. Hendershot, J. Forsaith in J. Pharmacol. Exptl. Ther. 125, 237 (1959), an der Maus untersucht. Dazu wurden männliche NMRI-Mäuse mit einem Gewicht zwischen 25 und 30 g verwendet. Je 10 Tiere erhielten pro Substanzdosis 30 Minuten nach oraler Gabe einer erfindungsgemäßen Verbindung 0,3 ml pro Maus einer 0,02 %igen wäßrigen Phenylchinon-Lösung (Phenylbenzoechinon, Fa. Sigma, Deisenhofen; Herstellung der Lösung unter Zusatz von 5 % Ethanol und Aufbewahrung im Wasserbad bei 45° C) intraperitoneal appliziert. Danach wurden die Tiere einzeln in Beobachtungskäfige gesetzt. Mit Hilfe eines Drucktastenzählers wurde 5 - 20 Minuten nach Phenylchinongabe die Anzahl der Schmerz-induzierten Streckbewegungen (Writhing-Reaktion = Durchdrücken des Körpers mit Abstrekken der Hinterextremitäten) gezählt. Aus der dosisabhängigen Abnahme der Writhing-Reaktion im Vergleich zu parallel untersuchten Mäusen, denen ausschließlich Phenylchinon appliziert wurde, wurden mittels Regressionsanalyse (Auswertungsprogramm Martens EDV-Service, Eckental) die ED₅₀-Werte (effektive Dosis mit 50 % iger Hemmung der Writhing-Reaktion) mit 95 % Vertrauensbereich berechnet. Alle untersuchten erfindungsgemäßen Verbindungen zeigten eine ausgeprägte analgetische Wirkung. Die Ergebnisse sind in der nachfolgenden Tabelle zusammengestellt.

**Tabelle:**

| Writhing-Hemmung | | | |
|---|---|---|---|
| **Beispiel** | **erfindungsgemäße Verbindung** | **ED**_{**50**} **[mg/kg per os]** | **%-Hemmung bei 21,5 mg/kg per os** |
| 1 | (**1**) | 5,8 | |
| 2 | (**-1**) | 22,3 | |
| 2 | (**+1**) | 1,1 | |
| 3 | (**2**) | 13,2 | |
| 4 | (**3**) | | -81,3 |
| 5 | (**4**) | 15,5 | |
| 6 | (**5**) | 8,3 | |
| 7 | (**6**) | 11,8 | |
| 8 | (**7**) | 27,3 | |
| 9 | (**8**) | 12,9 | |
| 10 | (**9**) | 12,8 | |
| 11 | (**10**) | 12,9 | |
| 13 | (**12**) | 19,9 | |
| 15 | (**14**) | 10,5 | |
| 16 | (**15**) | 3,8 | |
| 17 | (**+15**) | | -95,2 |
| 18 | (**16**) | | -100,0 |
| 19 | (**-16**) | 16,1 | |
| 19 | (**+16**) | 1,0 | |
| 20 | (**17**) | | -87,0 |
| 21 | (**-17**) | | -58,3 |
| 21 | (**+17**) | | -97,2 |
| 22 | (**+18**) | 15,7 | |
| 24 | (**+21**) | 1,9 | |

## Patentansprüche

1. 1-Phenyl-3-dimethylamino-propanverbindungen der Formel I 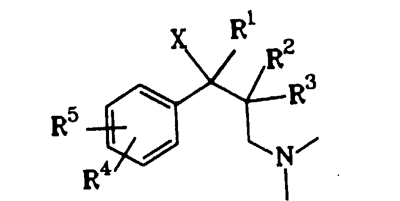 worin
X OH, F, Cl, H oder eine OCOR⁶-Gruppe mit R⁶ C₁₋₃-Alkyl bedeutet,
R¹ eine C₁₋₄-Alkylgruppe ist,
R² H oder eine C₁₋₄-Alkylgruppe und R³ H oder eine geradkettige C₁₋₄-Alkylgruppe bedeuten oder R² und R³ zusammen einen C₄₋₇-Cycloalkylrest bilden, und
wenn R⁵ H ist, R⁴ meta-O-Z bedeutet mit Z H, C₁₋₃-Alkyl, PO(O-C₁₋₄-Alkyl)₂, CO(OC₁₋₅-Alkyl), CONH-C₆H₄-(C₁₋₃-Alkyl), CO-C₆H₄-R⁷, wobei R⁷ ortho-OCOC₁₋₃-Alkyl oder meta- oder para-CH₂N(R⁸)₂ mit R⁸ C₁₋₄-Alkyl oder 4-Morpholino ist, oder R⁴ meta-S-C₁₋₃-Alkyl, meta-Cl, meta-F, meta-CR⁹R¹⁰R¹¹ mit R⁹, R¹⁰ und R¹¹ H oder F, ortho-OH, ortho-O-C₂₋₃-Alkyl, para-F oder para-CR⁹R¹⁰R¹¹ bedeutet, oder
wenn R⁵ para-ständiges Cl, F, OH oder O-C₁₋₃-Alkyl bedeutet, R⁴ meta-ständiges Cl, F, OH oder O-C₁₋₃-Alkyl bedeutet, oder
R⁴ und R⁵ zusammen 3,4-OCH=CH- oder 3,4-OCH=CHO- bedeuten,
als Diastereomere oder Enantiomere in Form ihrer Basen oder Salze von physiologisch verträglichen Säuren.

2. Verbindungen nach Anspruch 1, dadurch gekennzeichnet, daß X OH, F, Cl oder H darstellt, R¹ eine C₁₋₄-Alkylgruppe, R² H oder CH₃ und R³ H oder CH₃ bedeuten und
wenn R⁵ H ist, R⁴ meta-O-C₁₋₃-Alkyl, meta-OH, meta-S-C₁₋₃-Alkyl, meta-F, meta-Cl, meta-CH₃, meta-CF₂H, meta-CF₃ oder para-CF₃ bedeutet oder wenn R⁵ ein para-ständiges Cl oder F ist, R⁴ meta-ständiges Cl oder F bedeutet, oder
R⁴ und R⁵ zusammen 3,4-OCH=CH- bedeuten.

3. Verbindungen nach einem der Ansprüche 1 bis 2, dadurch gekennzeichnet, daß Verbindungen der Formel I, in der R² und R³ unterschiedliche Bedeutungen haben, in Form ihrer Diastereomeren mit der Konfiguration Ia 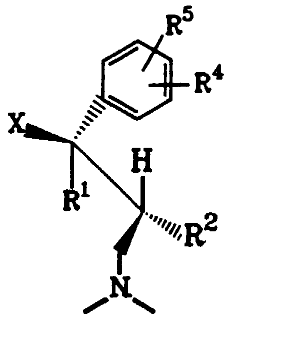 vorliegen.

4. Verfahren zur Herstellung von 1-Phenyl-3-dimethylamino-propanverbindungen der Formel I mit X = OH gemäß den Ansprüchen 1 bis 3, dadurch gekennzeichnet, daß man ein β-Dimethylaminoketon der Formel II 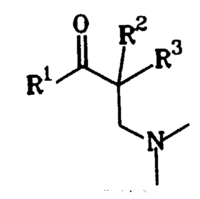 mit einer metallorganischen Verbindung der Formel III 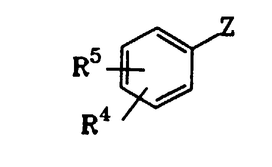 in der Z MgCl, MgBr, MgJ oder Li bedeutet, zu einer Verbindung der Formel I, in der X OH bedeutet, umsetzt.

5. Verfahren zur Herstellung von 1-Phenyl-3-dimethylamino-propanverbindungen der Formel I mit X = H gemäß den Ansprüchen 1 bis 3, dadurch gekennzeichnet, daß man eine Verbindung der Formel I, in der X Cl bedeutet, mit Zinkborhydrid, Zinkcyanoborhydrid und/oder Zinncyanoborhydrid umsetzt.

6. Verfahren zur Herstellung von 1-Phenyl-3-dimethylamino-propanverbindungen der Formel I mit X = F gemäß den Ansprüchen 1 bis 3, dadurch gekennzeichnet, daß man eine Verbindung der Formel 1 I, in der X OH bedeutet, mit Dimethylamino-schwefeltrifluorid in einem Lösungsmittel umsetzt.

7. Verfahren zur Herstellung von 1-Phenyl-3-dimethylamino-propanverbindungen der Formel I mit X = Cl gemäß Ansprüchen 1 bis 3, dadurch gekennzeichnet, daß man eine Verbindung der Formel I, in der X OH bedeutet, mit Thionylchlorid umsetzt.

8. Verfahren zur Herstellung von 1-Phenyl-3-dimethylamino-propanverbindungen der Formel I, in der X eine OCOR⁶⁻Gruppe mit R⁶ C₁₋₃-Alkyl bedeutet, gemäß Ansprüchen 1 bis 3, dadurch gekennzeichnet, daß man eine Verbindung der Formel I, in der X OH bedeutet, mit einem Säurechlorid Cl-COOR⁶ umsetzt.

9. Verwendung einer Verbindung gemäß den Ansprüchen 1 bis 3 zur Herstellung eines analgetischen Medikamentes.

10. Analgetikum, das als Wirkstoff wenigstens eine 1-Phenyl-3-dimethylamino-propanverbindung der Formel I gemäß den Ansprüchen 1 bis 3 enthält.

## Claims

1. 1-phenyl-3-dimethylamino-propane compounds of formula I 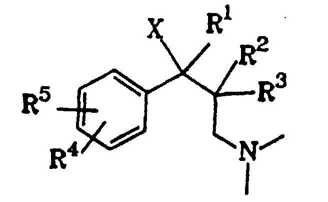 where
X represents OH, F, Cl, H or an OCOR⁶ group in which R⁶ is C₁₋₃-alkyl,
R¹ is a C₁₋₄-alkyl group,
R² represents H or a C₁₋₄-alkyl group and R³ represents H or a straight chain C₁₋₄-alkyl group or R² and R³ together form a C₄₋₇ cycloalkyl radical, and
when R⁵ is H, R⁴ represents meta-O-Z, where Z denotes H, C₁₋₃-alkyl, PO(O-C₁₋₄-alkyl)₂, CO(OC₁₋₅-alkyl), CONH-C₆H₄-(C₁₋₃-alkyl), CO-C₆H₄-R⁷, wherein R⁷ is ortho-OCOC₁₋₃-alkyl or meta- or para-CH₂N(R⁸)₂ in which R⁸ is C₁₋₄-Alkyl or 4-morpholino, or R⁴ represents meta-S-C₁₋₃-alkyl, meta-Cl, meta-F, meta-CR⁹R¹⁰OR¹¹ in which R⁹, R¹⁰ and R¹¹ represent H or F, ortho-OH, ortho-O-C₂₋₃-alkyl, para-F or para-CR⁹R¹⁰R¹¹, or
when R⁵ represents a para-Cl, F, OH or O-C₁₋₃-alkyl, R⁴ represents a meta Cl, F, OH or O-C₁₋₃-alkyl, or
R⁴ and R⁵ jointly represent 3,4-OCH=CH- or 3,4-OCH=CHO-,
as diastereoisomers or enantiomers in the form of their bases or salts of physiologically compatible acids.

2. Compounds according to claim 1, characterised in that X represents OH, F, Cl or H, R' represents a C₁₋₄-alkyl group, R² represents H or CH₃ and R³ represents H or CH₃, and
when R⁵ is H, R⁴ represents melta-O-C₁₋₃-alkyl, meta-OH, melta-S-C₁₋₃-alkyl, meta-F, meta-Cl, meta-CH₃, meta-CF₂H, meta-CF₃ or para-CF₃, or when R⁵ is a para-Cl or F, R⁴ represents a meta-Cl or F, or
R⁴ and R⁵ jointly represent 3,4-OCH= CH-.

3. Compounds according to either of claims 1 or 2, characterised in that compounds of formula I, in which R² and R³ have different meanings, are present in the form of their diastereoisomers with the configuration Ia

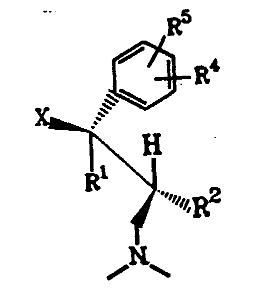

4. A method of preparing 1-phenyl-3-dimethylamino-propane compounds of formula I in which X = OH, according to claims 1 to 3, characterised in that a β-dimethylaminoketone of formula II 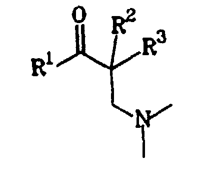 is reacted with an organometallic compound of formula III 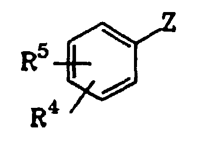 where Z represents MgCl, MgBr, MgI or Li, to form a compound of formula I in which X represents OH.

5. A method of preparing 1-phenyl-3-dimethylamino-propane compounds of formula I in which X = H, according to claims 1 to 3, characterised in that a compound of formula I, in which X represents Cl, is reacted with zinc borohydride, zinc cyanoborohydride and/or tin cyanoborohydride.

6. A method of preparing 1-phenyl-3-dimethylamino-propane compounds of formula I in which X = F, according to claims 1 to 3, characterised in that a compound of formula I, in which X represents OH, is reacted with dimethylamino-sulphur trifluoride in a solvent.

7. A method of preparing 1-phenyl-3-dimethylamino-propane compounds of formula I in which X = Cl, according to claims 1 to 3, characterised in that a compound of formula I, in which X represents OH, is reacted with thionyl chloride.

8. A method of preparing compounds of formula I, in which X represents an OCOR⁶ group in which R⁶ denotes C₁₋₃-alkyl, according to claims 1 to 3, characterised in that a compound of formula I in which X represents OH is reacted with an acid chloride Cl-COOR⁶.

9. The use of a compound according to claims 1 to 3 for the production of an analgesic drug.

10. An analgesic which contains, as its active ingredient, at least one 1-phenyl-3-dimethylamino-propane compound of formula I according to claims 1 to 3.

## Revendications

1. Composés de 1-phényl-3-diméthylaminopropane de formule I 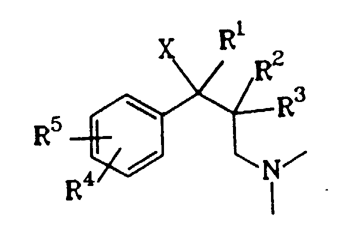 dans laquelle
X représente OH, F, Cl, H ou un groupe OCOR⁶ dans lequel R⁶ est un radical alkyle en C₁ à C₃,
R¹ est un groupe alkyle en C₁ à C₄,
R² représente H ou un groupe alkyle en C₁ à C₄ et R³ représente H ou un groupe alkyle linéaire en C₁ à C₄, ou bien R² et R³ forment ensemble un reste cycloalkyle en C₄ à C₇, et
lorsque R⁵ représente H, R⁴ désigne un groupe méta-O-Z dans lequel Z représente H, un groupe alkyle en C₁ à C₃, PO(O-alkyle en C₁ à C₄)₂, CO(O-alkyle en C₁ à C₅), CONH-C₆H₄-(alkyle en C₁ à C₃), CO-C₆H₄-R⁷ où R⁷ est un groupe ortho-OCOC-(alkyle en C₁ à C₃) ou méta- ou para-CH₂N(R⁸)₂ où R⁸ est un groupe alkyle en C₁ à C₄ ou 4-morpholino, ou bien R⁴ est un groupe métha-S-(alkyle en C₁ à C₃), méta-Cl, méta-F, méta-CR⁹R¹⁰R¹¹ où R⁹, R¹⁰ et R¹¹ représentent H ou F, un groupe ortho-OH, ortho-O-(alkyle en C₂ ou C₃), para-F ou para-CR⁹R¹⁰R¹¹, ou bien
lorsque R⁵ représente un radical Cl, F, OH ou O-(alkyle en C₁ à C₃) en position para, R⁴ représente un radical Cl, F, OH ou O-(alkyle en C₁ à C₃) en position méta, ou bien
R⁴ et R⁵ forment ensemble un groupe 3,4-OCH=CH- ou 3,4-OCH=CHO-,
à l'état de diastéréo-isomères ou d'énantiomères sous forme de leurs bases ou de sels d'acides acceptables du point de vue physiologique.

2. Composés suivant la revendication 1, caractérisés en ce que X représente OH, F, Cl ou H, R¹ est un groupe alkyle en C₁ à C₄, R² représente H ou CH₃ et R³ représente H ou CH₃,
lorsque R⁵ représente H, R⁴ est un groupe méta-O-alkyle en C₁ à C₃, méta-OH, méta-S-alkyle en C₁ à C₃, méta-F, méta-Cl, méta-CH₃, méta-CF₂H, méta-CF₃ ou para-CF₃ ou bien lorsque R⁵ représente Cl ou F en position para, R⁴ représente Cl ou F en position méta,
ou bien
R⁴ et R⁵ forment ensemble un groupe 3,4-OCH=CH-.

3. Composés suivant l'une des revendications 1 et 2, de formule I dans laquelle R² et R³ ont des définitions différentes, sous forme de leurs diastéréo-isomères ayant la configuration Ia

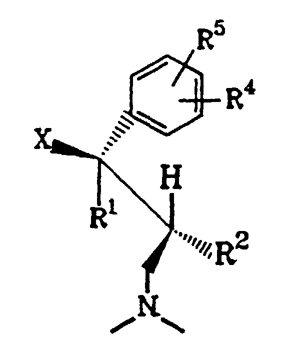

4. Procédés de production de composés de 1-phényl-3-diméthylaminopropane de formule I dans laquelle X représente OH suivant les revendications 1 à 3, caractérisés en ce qu'on fait réagir une β-diméthylaminocétone de formule II 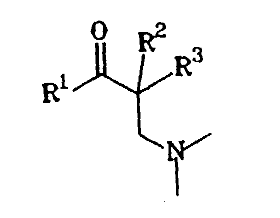 avec un composé organométallique de formule III 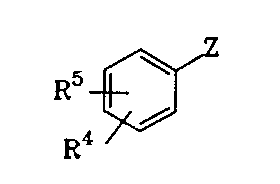 dans laquelle Z représente MgCl, MgBr, MgI ou Li, pour obtenir un composé de formule I dans laquelle X représente OH.

5. Procédé de production de composés de 1-phényl-3-diméthylaminopropane de formule I dans laquelle X représente H suivant les revendications 1 à 3, caractérisé en ce qu'on fait réagir un composé de formule I dans laquelle X représente Cl avec le borohydrure de zinc, le cyanoborohydrure de zinc et/ou le cyanoborohydrure d'étain.

6. Procédé de production de composés de 1-phényl-3-diméthylaminopropane de formule I dans laquelle X représente F suivant les revendications 1 à 3, caractérisé en ce qu'on fait réagir un composé de formule I dans laquelle X représente OH avec le trifluorure de diméthylaminosoufre dans un solvant.

7. Procédé de production de composés de 1-phényl-3-diméthylaminopropane de formule I dans laquelle X représente Cl suivant les revendications 1 à 3, caractérisé en ce qu'on fait réagir un composé de formule I dans laquelle X représente OH avec le chlorure de thionyle.

8. Procédé de production de composés de 1-phényl-3-diméthylaminopropane de formule I dans laquelle X représente un groupe OCOR⁶ où R⁶ est un groupe alkyle en C₁ à C₃, suivant les revendications 1 à 3, caractérisé en ce qu'on fait réagir un composé de formule I dans laquelle X représente OH avec un chlorure d'acide Cl-COOR⁶.

9. Utilisation d'un composé suivant les revendications 1 à 3 pour la préparation d'un médicament analgésique.

10. Analgésique qui contient comme substance active au moins un composé de 1-phényl-3-diméthylaminopropane de formule I suivant les revendications 1 à 3.
